# EUROPEAN PATENT APPLICATION

(11) **EP 4 627 937 A1**
(43) Date of publication of application: **08.10.2025**
(21) Application number: 23897836.5
(22) Date of filing: 29.11.2023
(51) Int. Cl.: A23L 33/135, A23L 2/52, A61K 8/99, A61K 35/747, A61P 1/02, A61Q 11/00

(54) **COMPOSITION FOR MAINTAINING HEALTH OF INTRAORAL ENVIRONMENT OR GUMS**

(30) Priority: 30.11.2022 JP 2022191773; 18.08.2023 JP 2023133266
(71) Applicant: Asahi Group Holdings, Ltd., Tokyo 130-8602 (JP)
(72) Inventor: SAKATA Shinji, Moriya-shi, Ibaraki 302-0106 (JP)
(74) Representative: J A Kemp LLP
(86) International application number: PCT/JP2023/042767
(87) International publication number: WO 2024/117190

(57) **Abstract**

The present invention provides a composition for maintaining an oral environment or gum health. Specifically, the composition contains, as an active ingredient, killed lactic acid bacteria that inhibit plaque maturation in an oral cavity.

## Description

### Technical Field

The present invention relates to an invention characterized by using killed lactic acid bacteria that inhibit plaque maturation in an oral cavity in order to maintain an oral environment or gum health.

### Background Art

The formation of dental plaque (also simply referred to as plaque) due to poor cleaning or the like is involved in deterioration of an oral environment including periodontal disease and others. The dental plaque, which is composed of oral bacteria and their products, starts to be formed when the oral bacteria adhere to tooth surfaces, and matures through coaggregation of the bacteria or the like while increasing the diversity of bacterial species. In addition, it is known that the composition of the bacterial community (bacterial flora) constituting the dental plaque changes during its maturation process (Non-Patent Literatures 7 to 10).

What is important to maintain an oral environment is not only professional plaque control by a dentist, but also personal plaque control (self-care).

As self-care methods, there are mechanical methods (cleaning with a toothbrush and the like) and chemical methods (use of a toothpaste, a mouthwash, and the like containing antibacterial agents).

However, the rate of plaque removed by toothbrushing is only about 40 to 50%, and a large quantity of plaque remains in the oral cavity even after cleaning.

In addition, under current legal regulations, the content of an antibacterial agent blended is prohibited from increasing to a level necessary for plaque control, so that a sufficient effect cannot be achieved.

Therefore, there is a demand for development for a self-care method effective to maintain an oral environment.

Lactic acid bacteria have been attracting attention as a food ingredient that can be used for self-care plaque control, and it is known that live or killed bacteria of specific species of lactic acid bacteria have abilities, such as inhibiting proliferation of oral pathogenic bacteria such as *Porphyromonas gingivalis,* which is considered as a periodontal pathogen, and *Fusobacterium nucleatum,* which is considered to be involved in periodontal disease (Patent Literatures 1 to 3 and Non Patent Literatures 1 to 6).

### Citation List

### Patent Literatures

Patent Literature 1: Published Japanese Translation of PCT International Application No. 2020-514309
Patent Literature 2: Japanese Patent Application Publication No. S61-91126
Patent Literature 3: Japanese Patent Application Publication No. S60-190707

### Non Patent Literatures

Non Patent Literature 1: Beneficial Microbes, 2018; 9 (4): 585-592
Non Patent Literature 2: Oral Health Prev Dent 2016; 14: 207-214
Non Patent Literature 3: J Clin Periodontol 2008; 35: 897-905
Non Patent Literature 4: J Periodontol. 2020; 91: 1328-1338
Non Patent Literature 5: AGING 2022, Vol. 14, No. 5, 2221-2238
Non Patent Literature 6: Letters in Applied Microbiology, 70, 310-317, 2020
Non Patent Literature 7: Japanese Journal of Lactic Acid Bacteria, Vol. 27, No. 1, 3-9
Non Patent Literature 8: Applied and Environmental Microbiology, Vol. 83, No. 11, e00407-17, 2017
Non Patent Literature 9: Clinical Oral Investigations, Vol. 21, 2399-2406, 2017
Non Patent Literature 10: Journal of Bacteriology, Vol. 187, No. 15 5330-5340, 2005

### Summary of Invention

### Problems to be solved by the invention

Live lactic acid bacteria remain in the oral cavity and produce lactic acid there, which poses a risk of acidic caries.

On the other hand, killed lactic acid bacteria have a small risk of acid caries, but their sufficient effect on the oral environment or gum health have not been found so far.

Therefore, an object is set to provide killed lactic acid bacteria capable of maintaining an oral environment or gum health.

As a result of carrying out intensive studies on this object, the present inventor found that "killed lactic acid bacteria" that inhibit plaque maturation are effective to maintain an oral environment or gum health. The present invention was made based on this finding.

Specifically, the present invention relates to the following [1] to [15].
[1] A composition for maintaining an oral environment or gum health, the composition comprising a killed lactic acid bacterium as an active ingredient, wherein
   the killed bacterium inhibits plaque maturation in an oral cavity.
[2] The composition according to the above [1], wherein the killed bacterium inhibits the plaque maturation by inhibiting proliferation of at least one species of oral bacteria selected from the group consisting of
   *Porphyromonas pasteri,*
   *Fusobacterium periodonticum,* and
   *Haemophilus parainfluenzae.*
[3] The composition according to the above [1] or [2], wherein the killed bacterium inhibits the plaque maturation by reducing an occupancy rate, in an oral bacterial flora, of at least one species of oral bacteria selected from the group consisting of:
   *Porphyromonas pasteri,*
   *Fusobacterium periodonticum,* and
   *Haemophilus parainfluenzae.*
[4] The composition according to any one of the above [1] to [3], wherein
   the killed bacterium is a killed lactic acid bacterium selected from the group consisting of
   the genus *Amylolactobacillus,*
   the genus *Furfurilactobacillus,*
   the genus *Lacticaseibacillus,*
   the genus *Lactiplantibacillus,*
   the genus *Latilactobacillus,*
   the genus *Lentilactobacillus,*
   the genus *Levilactobacillus,*
   the genus *Ligilactobacillus,*
   the genus *Limosilactobacillus,*
   the genus *Liquorilactobacillus,* and
   the genus *Paucilactobacillus.*
[5] The composition according to any one of the above [1] to [4], wherein
   the killed bacterium is a killed lactic acid bacterium selected from the group consisting of
   *Amylolactobacillus amylophilus,*
   *Furfurilactobacillus rossiae,*
   *Lacticaseibacillus casei,*
   *Lacticaseibacillus paracasei* subsp. *paracasei,*
   *Lacticaseibacillus paracasei* subsp. *tolerans,*
   *Lacticaseibacillus rhamnosus,*
   *Lacticaseibacillus zeae,*
   *Lactiplantibacillus plantarum,*
   *Latilactobacillus curvatus,*
   *Latilactobacillus sakei* subsp. *carnosus,*
   *Lentilactobacillus farraginis,*
   *Levilactobacillus brevis,*
   *Ligilactobacillus acidipiscis,*
   *Ligilactobacillus salivarius,*
   *Limosilactobacillus fermentum,*
   *Limosilactobacillus mucosae,*
   *Limosilactobacillus oris,*
   *Limosilactobacillus reuteri* subsp. *reuteri,*
   *Liquorilactobacillus oeni,* and
   *Paucilactobacillus kaifaensis.*
[6] The composition according to any one of the above [1] to [5], wherein
   the killed bacterium is a killed bacterium of *Ligilactobacillus salivarius.*
[7] The composition according to any one of the above [1] to [6], wherein
   the killed bacterium is a killed bacterium of a CP3365 strain (accession number: NITE BP-03781) of *Ligilactobacillus salivarius.*
[8] The composition according to any one of the above [1] to [7], wherein
   the maintaining the oral environment is inhibiting deterioration of plaque control record (PCR).
[9] The composition according to any one of the above [1] to [8], wherein
   the maintaining the gun health is inhibiting deterioration of probing pocket depth probing (PDP).
[10] The composition according to any one of the above [1] to [9], wherein
   the maintaining the gun health is inhibiting deterioration of bleeding on probing (BOP).
[11] The composition according to any one of the above [1] to [10], which is for a healthy individual.
[12] The composition according to any one of the above [1] to [11], which is a food and/or beverage composition.
[13] The composition according to the above [12], which is in a tablet form.
[14] The composition according to the above [12], which is in a beverage form.
[15] A CP3365 strain (accession number: NITE BP-03781) *of Ligilactobacillus salivarius* or a killed bacterium of the strain.

### Advantageous Effects of Invention

As will be described in Examples below, "killed lactic acid bacteria" that inhibit plaque maturation inhibit deterioration of an indicator of an oral environment or gum health. Using such killed bacteria as an active ingredient, the present invention is useful to maintain the oral environment or gum health.

### Brief Description of Drawings

Fig. 1 shows antibacterial activity of killed lactic acid bacteria against two species of oral bacteria (Fig. 1a: *Porphyromonas gingivalis* and Fig. 1b: *Fusobacterium nucleatum).*
Fig. 2 shows effects of killed bacteria of the CP3365 strain of lactic acid bacteria *Ligilactobacillus salivarius,* on PCR, BOP, and PPD which are indicators of an oral environment and gum health.
Fig. 3 shows stratified analysis results of the PCR, the BOP, and the PPD based on the presence or absence of *Porphyromonas gingivalis.*
Fig. 4 shows antibacterial activity of killed lactic acid bacteria against three species of oral bacteria (Fig. 4a: *Fusobacterium periodonticum,* Fig. 4b: *Porphyromonas pasteri,* and Fig. 4c: *Haemophilus parainfluenzae*).
Fig. 5 shows antibacterial activity of killed lactic acid bacteria against *Porphyromonas gingivalis.*
Fig. 6 shows antibacterial activity of killed lactic acid bacteria against *Fusobacterium nucleatum.*
Fig. 7 shows antibacterial activity of killed lactic acid bacteria against *Porphyromonas pasteri.*
Fig. 8 shows antibacterial activity of killed lactic acid bacteria against *Fusobacterium periodonticum.*
Fig. 9 shows antibacterial activity of killed lactic acid bacteria against *Haemophilus parainfluenzae.*

### Description of Embodiments

### [Lactic Acid Bacteria]

Lactic acid bacteria from which killed bacteria to be used in the present invention are derived are not particularly limited, as long as they are capable of inhibiting plaque maturation in an oral cavity, and examples thereof include the following lactic acid bacteria (1) to (12):
(1) the genus *Amylolactobacillus*;
(2) the genus *Furfurilactobacillus;*
(3) the genus *Lacticaseibacillus;*
(4) the genus *Lactiplantibacillus;*
(5) the genus *Lactobacillus;*
(6) the genus *Latilactobacillus;*
(7) the genus *Lentilactobacillus*;
(8) the genus *Levilactobacillus;*
(9) the genus *Ligilactobacillus;*
(10) the genus *Limosilactobacillus;*
(11) the genus *Liquorilactobacillus;* and
(12) the genus *Paucilactobacillus.*

The classification of lactic acid bacteria in this specification follows the reclassification published in 2020. Details of the reclassification are described in Int J Syst Evol Microbiol. 2020 Apr; 70(4): 2782-2858, Home Page of The Intestinal Microbiology Society (URL: https://bifidus-fund.jp/keyword/kw054.shtml), and the like.

Any known lactic acid bacterium that belongs to any of the above genera (1) to (12) and that inhibits plaque maturation in an oral cavity can be used without particular limitation in the present invention. Since the killed bacteria exert their effect through oral administration or intake, a strain whose safety has been confirmed in animals is preferred.

A preferable lactic acid bacterium is a lactic acid bacterium of (1) the genus *Amylolactobacillus,* (2) the genus *Furfurilactobacillus,* (3) the genus *Lacticaseibacillus,* (4) the genus *Lactiplantibacillus,* (6) the genus *Latilactobacillus,* (7) the genus *Lentilactobacillus,* (8) the genus *Levilactobacillus,* (9) the genus *Ligilactobacillus*, (10) the genus *Limosilactobacillus,* (11) the genus *Liquorilactobacillus,* or (12) the genus

### Paucilactobacillus.

A more preferable lactic acid bacterium is a lactic acid bacterium of (2) the genus *Furfurilactobacillus,* (3) the genus *Lacticaseibacillus,* (4) the genus *Lactiplantibacillus,* (6) the genus *Latilactobacillus,* (7) the genus *Lentilactobacillus,* (8) the genus *Levilactobacillus,* (9) the genus *Ligilactobacillus,* (10) the genus *Limosilactobacillus,* (11) the genus *Liquorilactobacillus,* or (12) the genus *Paucilactobacillus.*

A further preferable lactic acid bacterium is a lactic acid bacterium of (3) the genus *Lacticaseibacillus,* (4) the genus *Lactiplantibacillus,* (6) the genus *Latilactobacillus,* (7) the genus *Lentilactobacillus,* (8) the genus *Levilactobacillus,* (9) the genus *Ligilactobacillus,* (10) the genus *Limosilactobacillus,* or (11) the genus *Liquorilactobacillus.*

An even further preferable lactic acid bacterium is a lactic acid bacterium of (3) the genus *Lacticaseibacillus,* (9) the genus *Ligilactobacillus,* or (10) the genus *Limosilactobacillus.*

A particularly preferable lactic acid bacterium is a lactic acid bacterium of (9) the genus *Ligilactobacillus.*

### (1) Lactic Acid Bacteria of the Genus Amylolactobacillus

Examples of lactic acid bacteria of the genus *Amylolactobacillus* include the followings:
*Amylolactobacillus amylophilus;* and
*Amylolactobacillus amylotrophicus.*

A preferable lactic acid bacterium of the genus *Amylolactobacillus* is *Amylolactobacillus amylophilus.*

Any available strain of *Amylolactobacillus amylophilus* may be used, either the type strain or another strain (for example, JCM1125^{T} strain).

Any available strain of *Amylolactobacillus amylotrophicus* may be used, either the type strain or another strain (for example, JCM1124^{T} strain).

### (2) Lactic Acid Bacteria of the Genus Furfurilactobacillus

Examples of lactic acid bacteria of the genus *Furfurilactobacillus* are listed below:
*Furfurilactobacillus rossiae*;
*Furfurilactobacillus curtus*; and
*Furfurilactobacillus siliginis.*

A preferable lactic acid bacterium of the genus *Furfurilactobacillus* is *Furfurilactobacillus rossiae.*

Any available strain of *Furfurilactobacillus rossiae* may be used, either the type strain or another strain (for example, JCM16176^{T} strain).

Any available strain of *Furfurilactobacillus curtus* may be used, either the type strain or another strain (for example, JCM31185^{T} strain).

Any available strain of *Furfurilactobacillus siliginis* may be used, either the type strain or another strain (for example, JCM16155^{T} strain).

### (3) Lactic Acid Bacteria of the Genus Lacticaseibacillus

Examples of lactic acid bacteria of the genus *Lacticaseibacillus* are listed below:
*Lacticaseibacillus casei*;
*Lacticaseibacillus paracasei* subsp. *paracasei*;
*Lacticaseibacillus paracasei* subsp. *tolerans*;
*Lacticaseibacillus rhamnosus*; and
*Lacticaseibacillus zeae.*

A preferable lactic acid bacterium of the genus *Lacticaseibacillus* is *Lacticaseibacillus casei, Lacticaseibacillus paracasei* subsp. *paracasei, Lacticaseibacillus paracasei* subsp. *tolerans,* or *Lacticaseibacillus rhamnosus.*

A more preferable lactic acid bacterium of the genus *Lacticaseibacillus* is *Lacticaseibacillus casei, Lacticaseibacillus paracasei* subsp. *paracasei,* or *Lacticaseibacillus paracasei* subsp. *tolerans.*

Any available strain of *Lacticaseibacillus casei* may be used, either the type strain or another strain (for example, JCM1134⁷ strain).

Any available strain of *Lacticaseibacillus paracasei* subsp. *paracasei* may be used, either the type strain or another strain (for example, JCM8130^{T} strain).

Any available strain of *Lacticaseibacillus paracasei* subsp. *tolerans* may be used, either the type strain or another strain (for example, JCM1171^{T} strain).

Any available strain of *Lacticaseibacillus rhamnosus* may be used, either the type strain or another strain (for example, JCM1136^{T} strain).

Any available strain of *Lacticaseibacillus zeae* may be used, either the type strain or another strain (for example, JCM11302^{T} strain).

### (4) Lactic Acid Bacteria of the Genus Lactiplantibacillus

Examples of lactic acid bacteria of the genus *Lactiplantibacillus* are listed below:
*Lactiplantibacillus plantarum;*
*Lactiplantibacillus plantarum* subsp. *plantarum;* and
*Lactiplantibacillus pentosus.*

A preferable lactic acid bacterium of the genus *Lactiplantibacillus* is *Lactiplantibacillus plantarum* or *Lactiplantibacillus plantarum* subsp. *plantarum.*

A more preferable lactic acid bacterium of the genus *Lactiplantibacillus* is *Lactiplantibacillus plantarum.*

**In** an embodiment of the present invention, a lactic acid bacterium of the genus *Lactiplantibacillus* may be a lactic acid bacterium other than *Lactiplantibacillus plantarum.*

Any available strain of *Lactiplantibacillus plantarum* may be used, either the type strain or another strain (for example, JCM1100).

Any available strain of *Lactiplantibacillus plantarum* subsp. *plantarum* may be used, either the type strain or another strain (for example, JCM1149^{T} strain).

Any available strain of *Lactiplantibacillus pentosus* may be used, either the type strain or another strain (for example, JCM1558^{T} strain).

### (5) Lactic Acid Bacteria of the Genus Lactobacillus

Examples of lactic acid bacteria of the genus *Lactobacillus* are listed below:
*Lactobacillus acidophilus;*
*Lactobacillus amylovorus;*
*Lactobacillus delbrueckii* subsp. *bulgaricus;*
*Lactobacillus delbrueckii* subsp. *delbrueckii;*
*Lactobacillus delbrueckii* subsp. *lactis;*
*Lactobacillus gasseri;* and
*Lactobacillus helveticus.*

A preferable lactic acid bacterium of the genus *Lactobacillus* is *Lactobacillus delbrueckii* subsp. *bulgaricus, Lactobacillus delbrueckii* subsp. *delbrueckii, Lactobacillus delbrueckii* subsp. *lactis,* or *Lactobacillus helveticus.*

A more preferable lactic acid bacterium of the genus *Lactobacillus* is *Lactobacillus delbrueckii* subsp. *bulgaricus* or *Lactobacillus delbrueckii* subsp. *lactis.*

Any available strain of *Lactobacillus acidophilus* may be used, either the type strain or another strain (for example, JCM1132^{T} strain).

Any available strain of *Lactobacillus amylovorus* may be used, either the type strain or another strain (for example, JCM1126^{T} strain).

Any available strain of *Lactobacillus delbrueckii* subsp. *bulgaricus* may be used, either the type strain or another strain (for example, JCM1002⁷ strain).

Any available strain of *Lactobacillus delbrueckii* subsp. *delbrueckii* may be used, either the type strain or another strain (for example, JCM1012⁷ strain).

Any available strain of *Lactobacillus delbrueckii* subsp. *lactis* may be used, either the type strain or another strain (for example, JCM1248^{T} strain).

Any available strain of *Lactobacillus gasseri* may be used, either the type strain or another strain (for example, JCM1131^{T} strain).

Any available strain of *Lactobacillus helveticus* may be used, either the type strain or another strain (for example, JCM1120^{T} strain).

### (6) Lactic Acid Bacteria of the Genus Latilactobacillus

Examples of lactic acid bacteria of the genus *Latilactobacillus* are listed below:
*Latilactobacillus curvatus;*
*Latilactobacillus sakei* subsp. *carnosus;* and
*Latilactobacillus sakei* subsp. *sakei.*

A preferable lactic acid bacterium of the genus *Latilactobacillus* is *Latilactobacillus curvatus* or *Latilactobacillus sakei* subsp. *carnosus.*

Any available strain of *Latilactobacillus curvatus* may be used, either the type strain or another strain (for example, JCM1096^{T} strain).

Any available strain of *Latilactobacillus sakei* subsp. *carnosus* may be used, either the type strain or another strain (for example, JCM11031^{T} strain).

Any available strain of *Latilactobacillus sakei* subsp. *sakei* may be used, either the type strain or another strain (for example, JCM1157^{T} strain).

### (7) Lactic Acid Bacteria of the Genus Lentilactobacillus

Examples of lactic acid bacteria of the genus *Lentilactobacillus* are listed below:
*Lentilactobacillus farraginis*;
*Lentilactobacillus buchneri;* and
*Lentilactobacillus hilgardii.*

A preferable lactic acid bacterium of the genus *Lentilactobacillus* is *Lentilactobacillus farraginis.*

Any available strain of *Lentilactobacillus farraginis* may be used, either the type strain or another strain (for example, JCM14108^{T} strain).

Any available strain of *Lentilactobacillus buchneri* may be used, either the type strain or another strain (for example, JCM1115^{T} strain).

Any available strain of *Lentilactobacillus hilgardii* may be used, either the type strain or another strain (for example, JCM1155^{T} strain).

### (8) Lactic Acid Bacteria of the Genus Levilactobacillus

Examples of lactic acid bacteria of the genus *Levilactobacillus* are listed below:
*Levilactobacillus brevis;* and
*Levilactobacillus spicheri.*

A preferable lactic acid bacterium of the genus *Levilactobacillus* is *Levilactobacillus brevis.*

Any available strain of *Levilactobacillus brevis* may be used, either the type strain or another strain (for example, JCM1059^{T} strain).

Any available strain of *Levilactobacillus spicheri* may be used, either the type strain or another strain (for example, JCM15956^{T} strain).

### (9) Lactic Acid Bacteria of the Genus Ligilactobacillus

Examples of lactic acid bacteria of the genus *Ligilactobacillus* are listed below:
*Ligilactobacillus acidipiscis;*
*Ligilactobacillus salivarius;*
*Ligilactobacillus animalis;* and
*Ligilactobacillus agilis.*

A preferable lactic acid bacterium of the genus *Ligilactobacillus* is *Ligilactobacillus acidipiscis, Ligilactobacillus salivarius,* or *Ligilactobacillus animalis.*

A more preferable lactic acid bacterium of the genus *Ligilactobacillus* is *Ligilactobacillus acidipiscis* or *Ligilactobacillus salivarius.*

A particularly preferable lactic acid bacterium of the genus *Ligilactobacillus* is *Ligilactobacillus salivarius.*

Any available strain of *Ligilactobacillus acidipiscis* may be used, either the type strain or another strain (for example, JCM10692^{T} strain).

Any available strain of *Ligilactobacillus salivarius* may be used, either the type strain or another strain (for example, JCM1231^{T} strain, JCM1040 strain, JCM1150 strain, JCM7706 strain, or JCM7712 strain).

A particularly preferable *Ligilactobacillus salivarius* is the CP3365 strain. The CP3365 strain was submitted for an international deposit with the Patent Microorganisms Depositary of the National Institute of Technology and Evaluation (Room 122, 2-5-8 Kazusa Kamatari, Kisarazu City, Chiba Prefecture, Japan, Postal Code: 292-0818), an international depository authority as stipulated by the Budapest Treaty (identification on the receipt for the original deposit: CP3365. Receipt number: NITE ABP-03781. Date of receipt of the original deposit application: November 16, 2022. Accession number: NITE BP-03781).

Any available strain of *Ligilactobacillus animalis* may be used, either the type strain or another strain (for example, JCM5670^{T} strain).

Any available strain of *Ligilactobacillus agilis* may be used, either the type strain or another strain (for example, JCM1187^{T} strain).

### (10) Lactic Acid Bacteria of the Genus Limosilactobacillus

Examples of lactic acid bacteria of the genus *Limosilactobacillus* are listed below:
*Limosilactobacillus fermentum;*
*Limosilactobacillus mucosae;*
*Limosilactobacillus oris;*
*Limosilactobacillus reuteri* subsp. *reuteri;* and
*Limosilactobacillus reuteri.*

A preferable lactic acid bacterium of the genus *Limosilactobacillus* is *Limosilactobacillus fermentum, Limosilactobacillus mucosae, Limosilactobacillus oris,* or *Limosilactobacillus reuteri* subsp. *reuteri.*

A more preferable lactic acid bacterium of the genus *Limosilactobacillus* is *Limosilactobacillus mucosae* or *Limosilactobacillus reuteri* subsp. *reuteri.*

Any available strain of *Limosilactobacillus fermentum* may be used, either the type strain or another strain (for example, JCM1173^{T} strain).

Any available strain of *Limosilactobacillus mucosae* may be used, either the type strain or another strain (for example, JCM12515^{T} strain).

Any available strain of *Limosilactobacillus oris* may be used, either the type strain or another strain (for example, JCM11028^{T} strain).

Any available strain of *Limosilactobacillus reuteri* subsp. *reuteri* may be used, either the type strain or another strain (for example, JCM1112^{T} strain).

Any available strain of *Limosilactobacillus reuteri* may be used, either the type strain or another strain (for example, JCM1081^{T} strain).

### (11) Lactic Acid Bacteria of the Genus Liquorilactobacillus

Examples of lactic acid bacteria of the genus *Liquorilactobacillus* are listed below:
*Liquorilactobacillus oeni;* and
*Liquorilactobacillus mali.*

A preferable lactic acid bacterium of the genus *Liquorilactobacillus* is *Liquorilactobacillus oeni.*

Any available strain of *Liquorilactobacillus oeni* may be used, either the type strain or another strain (for example, JCM18036^{T} strain).

Any available strain of *Liquorilactobacillus mali* may be used, either the type strain or another strain (for example, JCM1116^{T} strain).

### (12) Lactic Acid Bacteria of the Genus Paucilactobacillus

Examples of lactic acid bacteria of the genus *Paucilactobacillus* are listed below:
*Paucilactobacillus kaifaensis;* and
*Paucilactobacillus hokkaidonensis.*

A preferable lactic acid bacterium of the genus *Paucilactobacillus* is *Paucilactobacillus kaifaensis.*

Any available strain of *Paucilactobacillus kaifaensis* may be used, either the type strain or another strain (for example, JCM33831^{T} strain).

Any available strain of *Paucilactobacillus hokkaidonensis* may be used, either the type strain or another strain (for example, JCM18461^{T} strain).

A mutant or derivative of any of the specific strains listed above may be also used as long as it inhibits plaque maturation in an oral cavity.

A preferable lactic acid bacterium is at least one species selected from the group consisting of:
(1) a lactic acid bacterium of the genus *Amylolactobacillus, Amylolactobacillus amylophilus;*
(2) a lactic acid bacterium of the genus *Furfurilactobacillus, Furfurilactobacillus rossiae;*
(3) lactic acid bacteria of the genus *Lacticaseibacillus,*
   *Lacticaseibacillus casei,*
   *Lacticaseibacillus paracasei* subsp. *paracasei,*
   *Lacticaseibacillus paracasei* subsp. *tolerans,*
   *Lacticaseibacillus rhamnosus,* and
   *Lacticaseibacillus zeae*;
(4) a lactic acid bacterium of the genus *Lactiplantibacillus,*
   *Lactiplantibacillus plantarum;*
(6) lactic acid bacteria of the genus *Latilactobacillus,*
   *Latilactobacillus curvatus,* and
   *Latilactobacillus sakei* subsp. *carnosus;*
(7) a lactic acid bacterium of the genus *Lentilactobacillus,*
   *Lentilactobacillus farraginis*;
(8) a lactic acid bacterium of the genus *Levilactobacillus,*
   *Levilactobacillus brevis;*
(9) lactic acid bacteria of the genus *Ligilactobacillus,*
   *Ligilactobacillus acidipiscis,* and
   *Ligilactobacillus salivarius;*
(10) lactic acid bacteria of the genus *Limosilactobacillus,*
   *Limosilactobacillus fermentum,*
   *Limosilactobacillus mucosae,*
   *Limosilactobacillus oris,* and
   *Limosilactobacillus reuteri* subsp. *reuteri;*
(11) a lactic acid bacterium of the genus *Liquorilactobacillus,*
   *Liquorilactobacillus oeni;* and
(12) a lactic acid bacterium of the genus *Paucilactobacillus,*
   *Paucilactobacillus kaifaensis.*

A more preferable lactic acid bacterium, a further preferable lactic acid bacterium, an even more preferable lactic acid bacterium, and a particularly preferable lactic acid bacterium are in the same ranges as those described regarding the genera of lactic acid bacteria.

From the viewpoint of antibacterial activity, a further preferable lactic acid bacterium is at least one species selected from the group consisting of:
(3) lactic acid bacteria of the genus *Lacticaseibacillus,*
   *Lacticaseibacillus casei,*
   *Lacticaseibacillus paracasei* subsp. *paracasei,*
   *Lacticaseibacillus paracasei* subsp. *tolerans,*
   *Lacticaseibacillus rhamnosus,* and
   *Lacticaseibacillus zeae*;
(9) a lactic acid bacterium of the genus *Ligilactobacillus,*
   *Ligilactobacillus salivarius;* and
(10) lactic acid bacteria of the genus *Limosilactobacillus,*
   *Limosilactobacillus fermentum,*
   *Limosilactobacillus mucosae,*
   *Limosilactobacillus oris,* and
   *Limosilactobacillus reuteri* subsp. *reuteri.*

A particularly preferable lactic acid bacterium is *Ligilactobacillus salivarius.*

A most preferable lactic acid bacterium is the CP3365 strain (accession number: NITE BP-03781) of *Ligilactobacillus salivarius.*

One species of lactic acid bacteria may be used alone or two or more species of lactic acid bacteria may be used in combination. Therefore, two or more species of killed lactic acid bacteria may be used in the present invention.

### [Killed Lactic Acid Bacteria]

A method for obtaining killed bacteria to be used in the present invention is not particularly limited, and any known sterilization method applicable to lactic acid bacteria may be used. As the sterilization method, there are a heat treatment, an ultraviolet light treatment, a radiation treatment (for example, such as gamma rays), a drug treatment (such as an antibiotic), and a chemical treatment (such as formalin). Among them, the heat treatment is preferred from the viewpoint of production efficiency.

For example, bacteria are collected from a culture of lactic acid bacteria, washed, suspended in water or the like, and then subjected to a heat treatment at 65 to 135°C (preferably 95 to 115°C) for 1 second to 45 minutes (preferably 20 to 40 minutes), so that the killed bacteria can be obtained.

The lactic acid bacteria to be used in the present invention can be cultured under common conditions for culturing lactic acid bacteria.

A culture medium is preferably a medium that contains a carbon source, a nitrogen source, inorganic salts, and so on, and that is capable of culturing lactic acid bacteria efficiently. The culture medium may be either a natural culture medium or a synthetic culture medium, and any known culture medium suitable for a stain to be used can be selected as appropriate.

As the carbon source, there are lactose, glucose, sucrose, fructose, galactose, blackstrap molasses, and so on.

The nitrogen source is an organic nitrogen-containing substance such as casein hydrolysate, whey protein hydrolysate, or soy protein hydrolysate.

The inorganic salts are phosphate, sodium, potassium, magnesium, and the like.

The culture media suitable for lactic acid bacteria include MRS medium, GLM medium, GAM medium, BL medium, Briggs Liver Broth, animal milk, skim milk, dairy whey, and the like, among which the MRS medium and the GLM medium are preferred.

Instead, in a case where killed lactic acid bacteria are used in food applications, a culture medium containing only food ingredients and food additives can be used.

Lactic acid bacteria can be cultured, for example, at temperature of 20 to 50°C (preferably 30 to 40°C) under a static or anaerobic condition. The culture temperature can be adjusted by using a thermostatic chamber, a mantle heater, a jacket, or the like.

The anaerobic condition refers to a low-oxygen environment with an oxygen level that allows lactic acid bacteria to grow. The anaerobic condition can be established by using an anaerobic chamber, an anaerobic box, a sealed container or bag containing an oxygen absorber, or the like.

The culture mode is static culture, agitation culture, tank culture, or the like.

The culture time is, for example, 3 to 96 hours, and preferably 12 to 32 hours.

The killed lactic acid bacteria may be in the form of a dried product (for example, a freeze-dried product), a crushed product of the dried product, and a treated product of killed bacteria such as an enzyme-treated product.

### [Inhibition of Plaque Maturation]

The killed lactic acid bacteria used in the present invention inhibit plaque maturation in an oral cavity. **In** other words, killed lactic acid bacteria that do not inhibit plaque maturation in an oral cavity are not included in the present invention.

The oral cavity is appropriately set depending on a target to which the present invention is to be applied, and is preferably of a mammal (human or non-human mammal), and more preferably of a human.

**In** the oral cavity, the plaque formation starts with adhesion of oral bacteria to the tooth surfaces. After that, the plaque matures while increasing the diversity of bacterial species through coaggregation of the oral bacteria and the like. Accordingly, "plaque maturation" may be recognized as "progress of plaque formation".

The "inhibition of plaque maturation" is caused by actions such as the following (1) and (2) exerted by the killed lactic acid bacteria.
(1) To inhibit the viability and proliferation ability of oral bacteria involved in the plaque maturation. In other words, the killed lactic acid bacteria have antibacterial activity against oral bacteria involved in the plaque maturation.
(2) To change a composition ratio (occupancy rate) of oral bacteria involved in the plaque maturation in an oral bacterial flora (for example, a flora in saliva or plaque). The action (2) includes reducing the occupancy rate of the oral bacteria involved in the plaque maturation in the oral bacterial flora, and suppressing an increase in the occupancy rate.

Examples of oral bacteria considered to be involved in the plaque formation include:
*Fusobacterium nucleatum;*
*Fusobacterium periodonticum;*
*Haemophilus parainfluenzae*;
*Porphyromonas gingivalis;* and
*Porphyromonas pasteri.*

In an embodiment of the present invention, oral bacteria which are involved in the plaque maturation are *Porphyromonas pasteri, Fusobacterium periodonticum,* and *Haemophilus parainfluenzae.* Specifically, in an embodiment of the present invention, targeting at least one species of oral bacteria selected from the group consisting of *Porphyromonas pasteri, Fusobacterium periodonticum,* and *Haemophilus parainfluenzae,* killed lactic acid bacteria inhibit plaque maturation by (1) reducing the number of and/or inhibiting the proliferation of the oral bacteria in an oral cavity, and/or (2) reducing the occupancy rate of the oral bacteria in an oral bacterial flora (preferably the flora in saliva or plaque) and/or suppressing an increase in the occupancy rate of the oral bacteria.

The detailed reasons why the plaque maturation in the oral cavity is inhibited with the aforementioned actions (1) and (2) exerted on at least one species of oral bacteria selected from the group consisting of *Porphyromonas pasteri, Fusobacterium periodonticum,* and *Haemophilus parainfluenzae* are unclear, but these bacteria may act as a bridge between early colonizers (such as bacteria that adhere to the tooth surfaces at the early stage of plaque formation) and late colonizers (such as bacteria that adhere at a stage where the number of bacterial species constituting the plaque increases rapidly in the plaque formation) (Non Patent Literature 7). For example, *Haemophilus parainfluenzae* coaggregates with streptococci that adhere to the tooth surfaces and plays a role at the early stage of the plaque formation (Non Patent Literature 8). Meanwhile, it was suggested that, since the plaque formation at the late stage progresses along with an increase in *Porphyromonas pasteri, Porphyromonas pasteri* may potentially act as a key factor in the plaque formation at the late stage, although the direct mechanism is unknown (Non Patent Literature 9). Moreover, *Fusobacterium periodonticum* is oral bacteria that proliferate at the later stage of the plaque formation, and it was demonstrated that *Fusobacterium periodonticum* has adhesins to be involved in coaggregation and thus is involved in plaque maturation (Non Patent Literature 10). The killed lactic acid bacteria of the present invention are considered to be capable of preventing plaque maturation by acting on these bacteria and inhibiting the plaque formation from progressing from the early stage to the late stage.

In an embodiment of the present invention, the killed lactic acid bacteria preferably exert the above actions (1) and/or (2) on *Porphyromonas pasteri,* and more preferably exert the above actions (1) and/or (2) on all of *Porphyromonas pasteri, Fusobacterium periodonticum,* and *Haemophilus parainfluenzae.*

### [Maintaining Oral Environment or Gum Health]

In the present invention, the killed lactic acid bacteria that inhibit the plaque maturation in an oral cavity are used for maintaining an oral environment or gum health.

Therefore, one aspect of the present invention is a composition for maintaining an oral environment or gum health, comprising, as an active ingredient, a killed lactic acid bacterium that inhibits plaque maturation in an oral cavity. Further, use of a killed lactic acid bacterium that inhibits plaque maturation in an oral cavity in order to prepare a composition for maintaining an oral environment or gum health is also one aspect of the present invention.

The phrase "maintaining an oral environment" is used as meaning including reducing oral bacteria involved in the plaque maturation and/or inhibiting proliferation of the oral bacteria, as well as changing the occupancy rate of the oral bacteria in an oral bacterial flora. The changing the occupancy rate of the oral bacteria in the oral bacterial flora includes reducing the occupancy rate of the oral bacteria in the oral bacterial flora and suppressing an increase in the occupancy rate.

The "oral environment" can be evaluated by using plaque control record (PCR) as an indicator. The PCR is commonly used in the dental field to indicate the state of plaque adhesion on teeth.

Therefore, the "maintaining an oral environment" includes inhibiting and/or mitigating deterioration of the PCR.

The "maintaining gum health" is used as meaning including inhibiting and/or mitigating gingival deterioration caused by plaque maturation.

The "gingival deterioration" can be evaluated by using probing pocket depth (PPD) and bleeding on probing (BOP) as indicators. Both of them are commonly used in the dental field, where the PPD indicates a periodontal pocket depth, and the BOP indicates bleeding during periodontal pocket measurement.

Therefore, the "maintaining gum health" includes inhibiting and/or mitigating deterioration of the PPD and/or the BOP.

### [Application Targets]

The composition of the present invention (hereinafter also referred to as "the composition") can be applied to any animals that need to maintain their oral environments or gum health without limitation. The application targets may be preferably mammals (humans and non-human mammals (for example, houses and cows)), and more preferably humans.

The application target may be a healthy subject (a healthy individual in the case of a human) or may be a subject having a deteriorated oral environment or gum health. In addition, an application target may be any one regardless of gender or age. In the present invention, the healthy individual means a person having a healthy oral environment, and means, for example, a person in whom the PPD (probing pocket depth) of 4 mm or more is 10% or less and the BOP (bleeding on probing) is 10% or less.

### [Optional Ingredients]

The composition may contain one or more optional ingredients as long as they will not impair the actions of the active ingredient. The optional ingredients may be selected as appropriate depending on a usage mode of the composition.

The optional ingredients may be additives used in foods and/or beverages, drugs, and the like.

Examples of the additives include: fats and oils (vegetable oils (such as soybean oil, corn oil, safflower oil, and olive oil) and animal fats (such as beef tallow and sardine oil)); herbal medicines (such as royal jelly and carrot); amino acids (such as glutamine, cysteine, leucine, and arginine); polyhydric alcohols (ethylene glycol, polyethylene glycol, propylene glycol, glycerin, and sugar alcohols (such as erythritol, xylitol, maltitol, and mannitol)); natural polymers (such as gum arabic, agar, water-soluble corn fiber, gelatin, xanthan gum, casein, gluten or gluten hydrolysate, lecithin, starch, and dextrin); vitamins (such as vitamin C and vitamin B group); minerals (such as calcium, magnesium, zinc, and iron); dietary fiber (such as mannan, pectin, and hemicellulose); surfactants (such as glycerin fatty acid ester and sorbitan fatty acid ester); purified water; excipients (such as maltodextrin, silicon dioxide, calcium stearate, hydroxypropyl cellulose, glucose, corn starch, and lactose); stabilizers; pH adjusters; antioxidants; sweeteners (such as sorbitol); taste ingredients; acidulants; colorings; flavorings; and others.

Further, the composition may optionally contain functional ingredients other than the active ingredient (killed lactic acid bacteria) (for example, taurine, glutathione, carnitine, creatine, coenzyme Q, glucuronic acid, glucuronolactone, chili pepper extract, ginger extract, cacao extract, guarana extract, garcinia extract, theanine, γ-aminobutyric acid, capsaicin, capsiate, various organic acids, flavonoids, polyphenols, catechins, xanthine derivatives, indigestible oligosaccharides such as fructooligosaccharides, polyvinylpyrrolidone, and others).

The optional ingredients are known substances which can be easily available on the market or be prepared.

One kind of optional ingredients may be used alone or two or more kinds of optional ingredients may be used in combination.

The content of each optional ingredient may be set as appropriate depending on the purpose of the addition and the like.

### [Food and/or Beverage Composition]

The killed lactic acid bacteria may be blended in foods and/or beverages. Therefore, one aspect of the present invention is a food and/or beverage composition for maintaining an oral environment or gum health.

The food and/or beverage includes all foods and beverages that can contain the killed lactic acid bacteria.

Specific examples of the foods and/or beverages include functional foods and/or beverages (including various forms such as tablets, chewable tablets, powders, capsules, granules, and drinks (beverages)); beverages (such as tea drinks (such as green tea, oolong tea, and black tea), soft drinks, jelly drinks, sports drinks, milk drinks, carbonated drinks, vegetable drinks, fruit juice drinks, fermented vegetable drinks, fermented fruit juice drinks, fermented milk drinks (such as yogurts), lactic acid bacteria drinks, milk drinks (such as coffee milk and fruit milk), powdered drinks, cocoa drinks, milk, and purified water); spreads (such as butters, jams, *furikake,* and margarines); egg products (such as mayonnaise); shortenings; custard creams; dressings; breads; rice; noodles; pastas; miso soups; tofu; dairy products (such as yogurts); soups or sauces; sweets (such as biscuits, cookies, chocolates, jellies, candies, cakes, ice creams, chewing gums, and tablets); breads, liquid foods, and others. Among them, the functional foods and beverages are preferred.

The functional food and/or beverage refers to a food and/or beverage that exerts the function of maintaining the oral environment or gum health with killed lactic acid bacteria inhibiting plaque maturation, and in other words, a food and/or beverage for maintaining the oral environment or gum health.

The functional food and/or beverage includes all types of so-called health foods and beverages. Examples of the functional foods and beverages include health functional foods (foods for specified health uses (including conditional foods for specified health uses), foods with functional claims, and nutritional functional foods), foods and beverages for special dietary uses, nutritional supplemental foods and beverages, health supplemental foods and beverages, supplements (including various dosage forms such as tablets, coated tablets, sugar-coated tablets, capsules, and liquids), foods and beverages for beauty (such as diet foods and beverages), and others.

The functional food and/or beverage includes health foods and beverages with health claims based on the Codex Alimentarius (of the FAO/WHO Joint Codex Alimentarius Commission).

In addition to the killed lactic acid bacteria, the food and/or beverage may optionally contain additives permitted for incorporation into foods and beverages.

Examples of the additives include color formers (such as sodium nitrite); coloring agents (such as gardenia pigment and red 102); flavorings (such as orange flavoring); sweeteners (such as stevia and aspartame); preservatives (such as sodium acetate and sorbic acid); emulsifiers (such as sodium chondroitin sulfate and propylene glycol fatty acid esters); antioxidants (such as disodium EDTA and vitamin C); pH adjusters (such as citric acid); chemical seasonings (such as sodium inosinate); thickeners (such as xanthan gum); leavening agents (such as calcium carbonate); antifoaming agents (calcium phosphate); binding agents (such as sodium polyphosphate); nutritional supplements (such as calcium supplement and vitamin A); excipients (such as water-soluble dextrin), and so on.

The food and/or beverage composition containing the killed lactic acid bacteria can be manufactured by using means available in the food industry.

For example, the killed bacteria prepared in a liquid, gel, solid, powder or granule form may be added to a food and/or beverage. The killed bacteria may be directly mixed, dissolved, or suspended in raw materials for a food and/or beverage.

The killed bacteria may be applied to, coated on, permeated into, or sprayed on a food and/or beverage.

The killed bacteria may be uniformity distributed or localized in a food and/or beverage.

In the case of a functional food and/or beverage, the killed bacteria may be enclosed in a capsule, an edible film, an edible coating agent, or the like.

In addition, in the case of a functional food and/or beverage, an appropriate excipient or the like may be first added to the killed bacteria, and then the obtained mixture may be formed into a shape such as a tablet. For tableting, the killed bacteria may be mixed with an excipient, a stabilizer, a flavoring, and others which are permitted for manufacturing, as needed, and then the obtained mixture may be tableted according to the standard method. In addition, for tableting, the killed bacteria may be mixed with an excipient, a binder, a disintegrant, a lubricant, a flavoring agent, a suspending agent, a coating agent, and other optional ingredients, as long as they will not impair the actions.

The shape of the functional food and/or beverage may be a tablet, a pill, a capsule, a granule, a powder, a syrup, or the like, and oral administration thereof is desirable.

The food and/or beverage containing the killed bacteria may be further processed and such processed products may be included in the scope of the present invention.

The content of the killed bacteria in the food and/or beverage composition may be set as appropriate with the form of the food and/or beverage, an intake interval, and so on taken into consideration. **In** the case of tablets, the content of the killed bacteria per 1 g of the tablets (the number of bacterial cells) is, for example, 8.0×10⁸ to 3.0×10¹⁰ cells/g, and preferably 4.0×10⁹ to 9.5×10⁹ cells/g.

In the case where the killed bacteria are taken as a food and/or beverage composition, the number of killed bacteria to be taken per day by an administration target is, for example, 2.0×10⁹ to 2.0×10¹¹ cells and preferably 3.0×10⁹ to 1.3×10¹¹ cells.

The intake interval is not particularly limited and may be set as appropriate depending on a type of food and/or beverage and others, such as once a day or several times a day.

An intake period is also not particularly limited and may be set as appropriate depending on a type of food and/or beverage and others.

### [Pharmaceutical Composition]

The killed lactic acid bacteria that inhibit plaque maturation in an oral cavity may be used as an active ingredient for pharmaceuticals or quasi-pharmaceutical products. Therefore, one aspect of the present invention is a pharmaceutical composition for maintaining an oral environment or gum health. Further, use of the killed lactic acid bacteria that inhibit plaque maturation in an oral cavity in order to prepare the pharmaceutical composition is also one aspect of the present invention.

The pharmaceutical compositions include not only pharmaceuticals but also quasi-pharmaceutical products (for example, oral care products such as toothpastes and mouthwashes).

A dosage form of the pharmaceutical composition is not particularly limited. Examples of the dosage form include oral preparations (such as tablets, capsules, granules, powders, syrups, dry syrups, liquids, suspensions, and films) and others.

A liquid preparation such as a liquid medicine or a suspension may be prepared immediately before administration by dissolving or suspending the pharmaceutical composition in water or another suitable medium.

Tablets, granules, or the like may be surface-coated in a known method.

The pharmaceutical composition may be a sustained-release, delayed-release or immediate-release preparation produced according to a known controlled release technique.

The pharmaceutical composition may optionally contain a pharmaceutically acceptable carrier and/or additive.

Examples of the pharmaceutically acceptable carrier and/or additive include water, organic solvent, collagen, polyvinyl alcohol, polyvinylpyrrolidone, carboxyvinyl polymer, sodium alginate, water-soluble dextran, water-soluble dextrin, sodium carboxymethyl starch, pectin, xanthan gum, gum arabic, casein, gelatin, agar, glycerin, propylene glycol, polyethylene glycol, petrolatum, paraffin, stearyl alcohol, stearic acid, human serum albumin, mannitol, sorbitol, lactose, surfactant, and artificial cell structure (such as liposome), and the like.

The pharmaceutical composition may be applied to any animal needing to maintain its oral environment or gum health without limitation. The application targets may be preferably mammals (humans and non-human mammals (for example, houses and cows)), and more preferably humans. In addition, an application target may be any one regardless of gender or age.

The dosage of the pharmaceutical composition may be set as appropriate depending on the body weight of an administration target, the number of administrations, and so on.

In the case of oral administration, the number of killed bacterial cells to be administered to a target per day is, for example, 2.0×10⁹ to 2.0×10¹¹ cells and preferably 3.0×10⁹ to 1.3×10¹¹ cells.

An administration interval may be set as appropriate such as once a day or several times a day.

### [Feed Composition]

The killed lactic acid bacteria can be incorporated into animal feed. Therefore, one aspect of the present invention is a feed composition for maintaining an oral environment or gum health.

Examples of the feed include feed for livestock (such as cattle and pigs), race horses, and pets (such as dogs and cats).

Since the feed is almost the same as the foods and/or beverages except that the targets are other than humans, the above description concerning the food and/or beverage composition also applies to the feed composition.

### [Examples]

Next, specific effects of the present invention will be specifically described by using Examples, but the present invention should not be limited to Examples.

### [Test Strains]

Lactic acid bacteria strains and oral bacteria strains specified in Table 1 were used. In Table 1, T in a strain number indicates the type strain.

Except for the CP3365 strain (accession number: NITE BP-03781) of *Ligilactobacillus salivarius,* for which the international deposit procedure was carried out, the bacteria strains were provided by the depository institutions listed in Table 1.

### [Culture of Lactic Acid Bacteria and Preparation of Killed Bacteria]

According to the following procedure, each lactic acid bacteria strain was cultured and subsequently killed bacteria were prepared.

The lactic acid bacteria strain was cultured in a culture medium (lactobacillus Man-Rogosa-Sharpe (MRS) agar (Difco, USA)) under an anaerobic condition using Anaeropack Kenki (MITSUBISHI GAS CHEMICAL COMPANY, INC., Japan) at 37°C for 24 hours.

The obtained colonies were inoculated into a culture medium (MRS broth (Difco, USA)) and cultured under an anaerobic condition at 37°C for 18 hours.

The cultivated medium was centrifuged at 3,000 rpm for 15 minutes to collect the bacteria. The collected bacteria were suspended in 0.75% NaCl physiological saline, further washed, and then collected. The quantity of bacteria collected (mg) from each lactic acid bacteria strain is shown in Table 1.

The collected bacteria were suspended in distilled water (D.W.) and subjected to a heat treatment at 105°C for 30 minutes to prepare killed bacteria. The killed bacteria were freeze-dried and stored until various tests to be described below.

### [Culture of Oral Bacteria]

Three strains, *Porphyromonas gingivalis, Fusobacterium nucleatum* and *Fusobacterium periodonticum,* were each cultured under an anaerobic condition at 37°C for 48 hours using a modified Gifu Anaerobic Medium (GAM) agar medium (Nissui Pharmaceutical Co., Ltd., Japan). The obtained colonies were inoculated into a modified GAM liquid culture medium (Nissui Pharmaceutical Co., Ltd., Japan) and cultured under an anaerobic condition at 37°C for 48 hours.

*Porphyromonas pasteri* was cultured in an ABHK culture medium (Nissui Pharmaceutical Co., Ltd., Japan) under an anaerobic condition at 37°C for 48 hours. The obtained colonies were inoculated into Entrched BHI broth [3.7% brain-heart infusion (Nissui Pharmaceutical Co., Ltd., Japan), 0.5% yeast extract (Difco, USA), and 0.05% cysteine (Fujifilm Wako Pure Chemical Industries, Ltd., Japan)] supplemented with 5 µg/ml of hemin (NACALAI TESQUE, INC., Japan) and 0.5 µg/ml menadione (NACALAI TESQUE, INC., Japan) and cultured under an anaerobic condition at 37°C for 48 hours.

*Haemophilus parainfluenzae* was cultured in Tripticase Soy agar (Difco, USA) supplemented with 10% defibrinated horse blood (Japan Bioserum, Japan) under an aerobic condition at 37°C for 24 hours. Subsequently, the colonies were inoculated into BHI broth (Nissui Pharmaceutical Co., Ltd., Japan) supplemented with 2 µg/ml of nicotinamide adenine dinucleotide (NACALAI TESQUE, INC., Japan) and cultured under an aerobic condition at 37°C for 24 hours.

### [Example 1: Evaluation of Antibacterial Activity of Killed Lactic Acid Bacteria Against Oral Bacteria (No. 1)]

The antibacterial activity of the killed lactic acid bacteria *(Ligilactobacillus salivarius)* against two species of oral bacteria *(Porphyromonas gingivalis* and *Fusobacterium nucleatum)* was evaluated. The evaluation was carried out by using the Microbial Viability Assay Kit (DOJINDO LABORATORIES, Japan) according to the provider's protocol as follows.

First, the killed lactic acid bacteria were suspended in 0.75% NaCl physiological saline to prepare a suspension (at a concentration of the killed bacteria: 0.5 mg/ml).

Separately from this, the oral bacteria were suspended in physiological saline to prepare a suspension with an optical density (OD600) of 0.1.

Next, 0.5 ml of the suspension of the killed bacteria and 4.5 ml of the suspension of the oral bacteria were mixed to adjust a final concentration of the lactic acid bacteria to 0.05 mg/ml, followed by incubation at 37°C for 30 minutes. After the incubation, 180 µl of the mixed suspension and 10 µl of WST-8 test solution (Dojindo, Japan) were added to each well of a 96-well plate and mixed.

The 96-well plate was immediately subjected to incubation at 37°C, and OD460 was measured using MULTISKAN GO (Thermo SCIENTIFIC, USA) at 0 minutes and 120 minutes after the start of the incubation.

In this evaluation system, the OD460 value reflects the respiratory activity of the oral bacteria, which means that a change in the OD460 value due to the incubation (ΔOD460 (120 minutes - 0 minutes)) is associated with a change in the viability (or proliferation ability) of the oral bacteria.

In a control, physiological saline was used in place of the killed lactic acid bacteria.

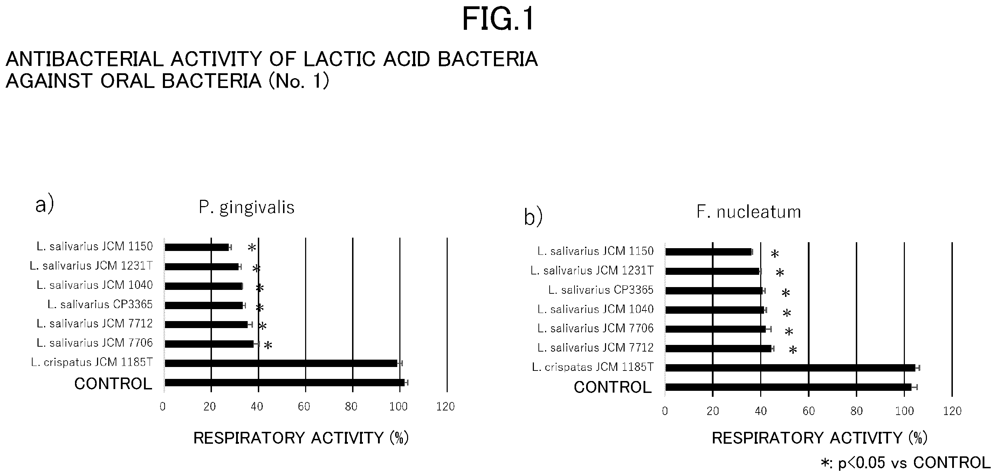

Fig. 1 shows the respiratory activity of the oral bacteria as a result of the addition of the killed bacteria of each lactic acid bacteria strain with the control set as a reference (100%).

The killed bacteria of *Lactobacillus crispatus* used as a comparative example did not demonstrate significant inhibition of the respiratory activity of the oral bacteria (Figs. 1a and 1b).

On the other hand, the killed bacteria of all the *Ligilactobacillus salivarius* strains significantly reduced the respiratory activity of the oral bacteria, *Porphyromonas gingivalis* to less than 40% (Fig. 1a) and significantly reduced the respiratory activity of the oral bacteria, *Fusobacterium nucleatum* to less than 45% (Fig. 1b).

Accordingly, it was demonstrated that the killed bacteria of *Ligilactobacillus salivarius* have antibacterial activity against *Porphyromonas gingivalis* and *Fusobacterium nucleatum.*

Of the six *Ligilactobacillus salivarius* strains whose antibacterial activity was confirmed, the CP3365 strain, from which the largest quantity of bacteria was collected, was subjected to further evaluation.

### [Example 2: Clinical Trial on Maintaining Oral Environment or Gum Health by Killed Lactic Acid Bacteria]

A clinical trial was conducted using a food (tablets) containing the killed lactic acid bacteria.

### [Test Food]

A test food was a tablet containing the killed bacteria of the CP3365 strain of *Ligilactobacillus salivarius* as an active ingredient and also containing a sweetener (sorbitol) and excipients (maltodextrin, fine powder of silicon dioxide, calcium stearate, and hydroxypropyl cellulose). The content of the killed lactic acid bacteria (number of cells) per 1 g of the tablets was 6.3×10⁹ cells/g .

Tablets in which crystalline cellulose was contained in place of the killed lactic acid bacteria were used as a control food.

### [Experiment Procedure]

As the present trial, a randomized, double-blind, placebo-controlled, parallel group comparison trial was carried out.

The subjects were persons each having a healthy oral environment (the PPD (probing pocket depth) of 4 mm or more is 10% or less and the BOP (bleeding on probing) is 10% or less).

The subjects were divided into two groups each including 32 subjects and the trial was carried out.

Starting the day after the start of the trial, the test group (CP3365 group) took the test food containing 1.3×10¹⁰ cells of the killed lactic acid bacteria per administration three times a day after meals.

Starting the day after the start of the trial, the control food group took the control food three times a day after meals.

The test and control foods were taken for 8 weeks.

### [Oral Cavity Examination]

The subjects were examined in terms of the PCR, the BOP, and the PPD.

The examinations were conducted before the intake and at week 4 and week 8 after the start of the intake. The subjects were instructed to refrain from brushing their teeth for two days prior to the examination in order to level their oral environments.

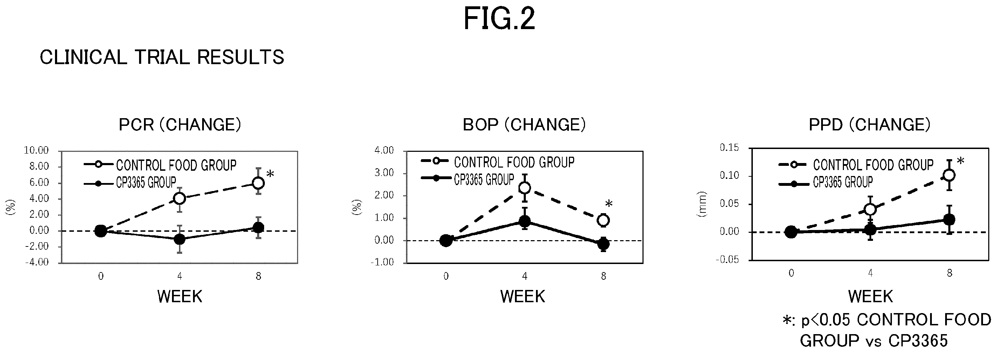

The PCR, the BOP, and the PPD were measured on all the teeth. The PCR was measured in accordance with the method of O'Leary, Dark & Naylor. The BOP and the PPD were measured in the method of four points for each tooth. The BOP was measured 30 seconds after the probing. Fig. 2 and Table 2 show the results expressed by amounts of changes based on the measured values before the intake.

In the control food group, the PCR, the BOP, and the PPD significantly increased at week 8.

On the other hand, in the test group, no significant increases were observed in all the PCR, the BOP, and the PPD.

To compare the two groups, the PCR of the control food group at week 4 was significantly higher than that of the test group. Moreover, all the PCR, the BOP, and the PPD of the control food group at week 8 were significantly higher than those of the test group.

As described above, the killed lactic acid bacteria of the CP3365 strain of *Ligilactobacillus salivarius* inhibited the deterioration of the PCR, the BOP and the PPD, demonstrating that they inhibited the plaque maturation in the oral cavity and maintained the oral environment and gum health.

### [Oral Bacterial Flora Analysis]

The bacterial floras of the subjects' saliva and supragingival plaque (hereafter referred to as plaque) were analyzed before the intake (week 0) and at week 8 after the start of the intake.

The saliva (1 ml or more) was collected without stimulation. The plaque was collected by wiping the buccal surfaces of all the molars with a cotton swab. The collected samples were stored at -20°C until DNA extraction.

The DNA extracted according to a usual manner was subjected to bacterial flora analysis at Genome Read Co., Ltd. using the meta-16S rRNA method.

In the bacterial flora analysis, a change over time of the occupancy rate of each of the following six species of oral bacteria in each of the salvia and plaque bacterial floras was evaluated. The results are presented in Table 3.
*Porphyromonas gingivalis*
*Porphyromonas pasteri*
*Fusobacterium nucleatum* subsp. *animalis*
*Fusobacterium nucleatum* subsp. *vincentii*
*Fusobacterium periodonticum*
*Haemophilus parainfluenzae*

In the plaque bacterial flora, the change in the occupancy rate of *Porphyromonas pasteri* in the test group (CP3365 group) at week 8 was marginally significantly reduced as compared to the control food group (Table 3a).

In the saliva bacterial flora, the occupancy rates of *Fusobacterium periodonticum* and *Haemophilus parainfluenzae* in the test group were significantly reduced as compared to the control food group (Table 3b).

Regarding *Fusobacterium nucleatum,* the subspecies *Fusobacterium nucleatum* subsp. *animalis* and *Fusobacterium nucleatum* subsp. *vincentii* were detected, but *Fusobacterium nucleatum* was not detected.

Regarding *Porphyromonas gingivalis,* the detection counts at week 8 were n=1 (the control food group) and n=3 (the test group) in the plaque, and were n=6 (the control food group) and n=10 (the test group) in the saliva, indicating that the proportion of the subjects in whom *Porphyromonas gingivalis* was detected was low. Regarding the change in the occupancy rate of *Porphyromonas gingivalis,* no significant difference was observed between the test group and the control group in either the plaque or the saliva.

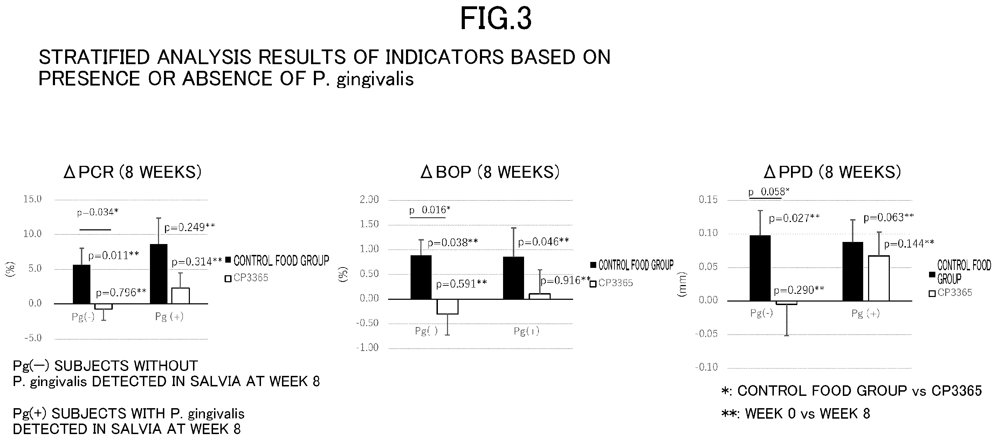

Next, in order to examine influences of *Porphyromonas gingivalis* (hereinafter abbreviated as Pg) on the oral environment or gum health (the PCR, the BOP, and the PPD), a stratified analysis was made based on the presence or absence of Pg. The subjects were stratified based on the presence or absence of Pg in their saliva at week 8 (Table 3b). Fig. 3 shows the results.

In the stratum (Pg(-)) in which Pg was not detected, the PCR, the BOP, and the PPD in the control food group all significantly increased at week 8 as compared to before the intake. In the stratum in which Pg was detected (Pg(+)), the control food group significantly increased the BOP and marginally significantly increased the PPD at week 8 as compared to before the intake. These results suggest that oral bacteria other than Pg are involved in the plaque maturation and the deterioration of the oral environment and gum health.

In the Pg(-) stratum, the changes in the PCR and the BOP in the control food group at week 8 were significantly increased as compared to the test group, and the change in the PPD in the control food group at week 8 was marginally significantly increased as compared to the test group.

On the other hand, in the Pg(+) stratum, no significant group difference between the control food group and the test group was not observed because the detection counts (n) were small, where n=6 (the control food group) and n=10 (the test group). However, in the control food group, the BOP was significantly increased and the PPD marginally was significantly increased at week 8 as compared to before the intake. In contrast to this, in the test group, such increases were not observed.

The above results demonstrate that the killed lactic acid bacteria of the CP3365 strain of *Ligilactobacillus salivarius* inhibited the proliferation of the three species of oral bacteria *(Porphyromonas pasteri, Fusobacterium periodonticum,* and *Haemophilus parainfluenzae*) and achieved reductions or inhibited increases in the occupancy rates of these oral bacteria in the oral bacterial floras. In addition, it was demonstrated that, regardless of the presence or absence of Pg, all the PCR, the BOP, and the PPD of the control food group were increased at week 8, or in other words, the plaque maturation was progressed and the oral environment or gum health was deteriorated, but that the killed bacteria of the CP3365 strain inhibited the increases in the PCR, the BOP, and the PPD, in other words, inhibited the plaque maturation and maintained the oral environment or gum health.

### [Example 3: Evaluation of Antibacterial Activity of Killed Lactic Acid Bacteria Against Oral Bacteria (No. 2)]

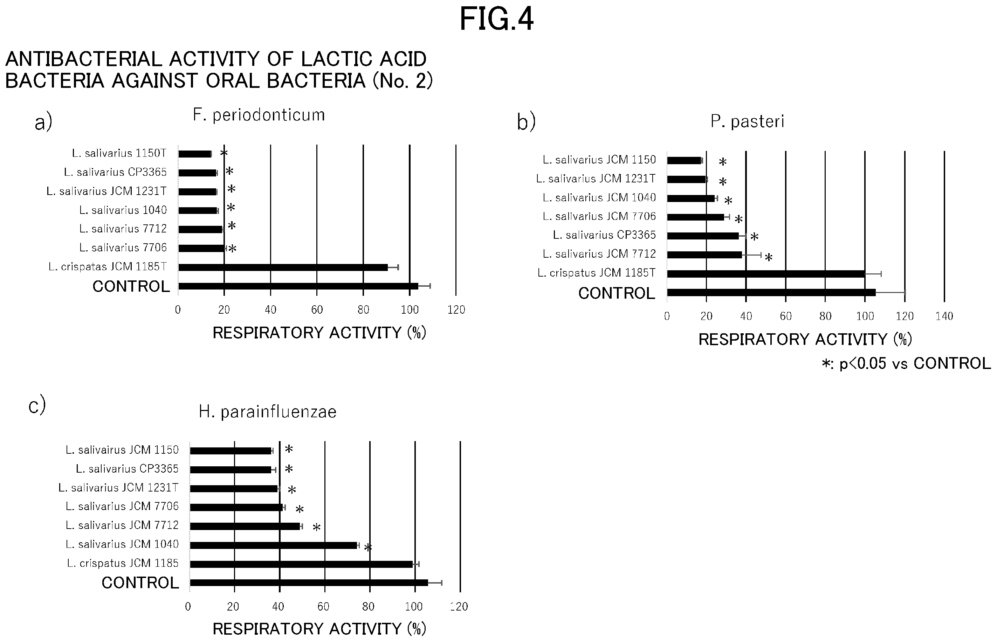

The antibacterial activity of the killed bacteria of each of the *Ligilactobacillus salivarius* strains other than the CP3365 strain against the three species of oral bacteria that were observed to have the reductions or inhibited increases in the occupancy rates in the oral floras in Example 2 was evaluated according to the same procedure as in Example 1. The antibacterial activity of the CP3365 strain and *Lactobacillus crispatus* were also evaluated. Fig. 4 shows the results.

The killed bacteria of *Lactobacillus crispatus* used as the comparative example demonstrated no significant inhibition of the respiratory activity of the oral bacteria (Figs. 4a to 4c).

On the other hand, the killed bacteria of the six species of *Ligilactobacillus salivarius* strains including the CP3365 strain significantly reduced the respiratory activity of *Fusobacterium periodonticum* to less than 20% (Fig. 4a), significantly reduced the respiratory activity of *Porphyromonas pasteri* to less than 40% (Fig. 4b), and significantly reduced the respiratory activity of *Haemophilus parainfluenzae* to less than 80% (Fig. 4c).

Therefore, it was demonstrated that the killed bacteria of the *Ligilactobacillus salivarius* strains also have the antibacterial activity against the three species of the oral bacteria targeted for the evaluation.

### [Example 4: Evaluation of Antibacterial Activity of Killed Lactic Acid Bacteria Against Oral Bacteria (No. 3)]

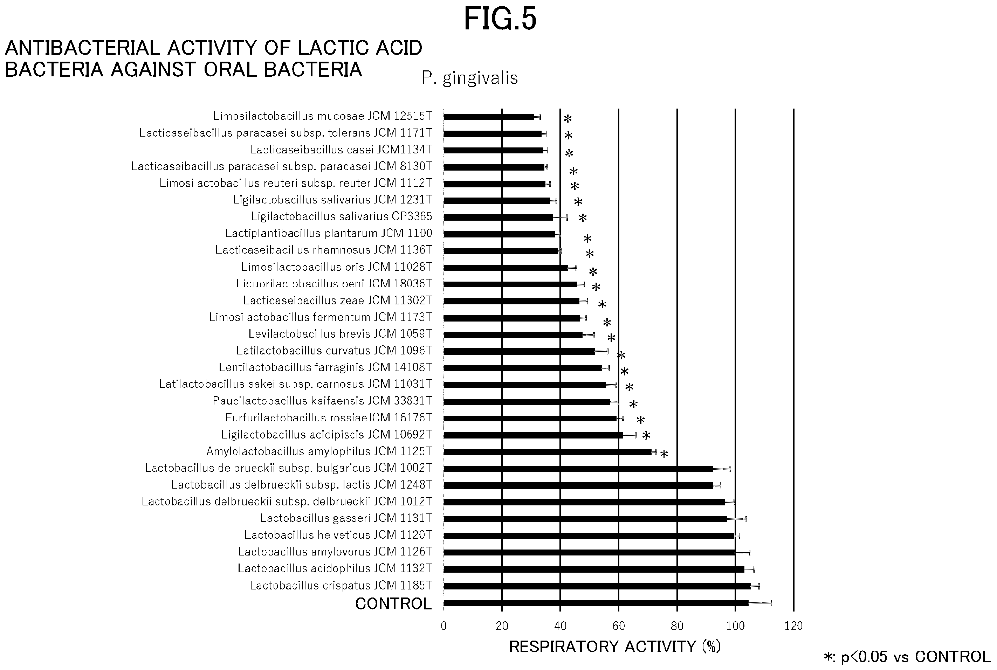

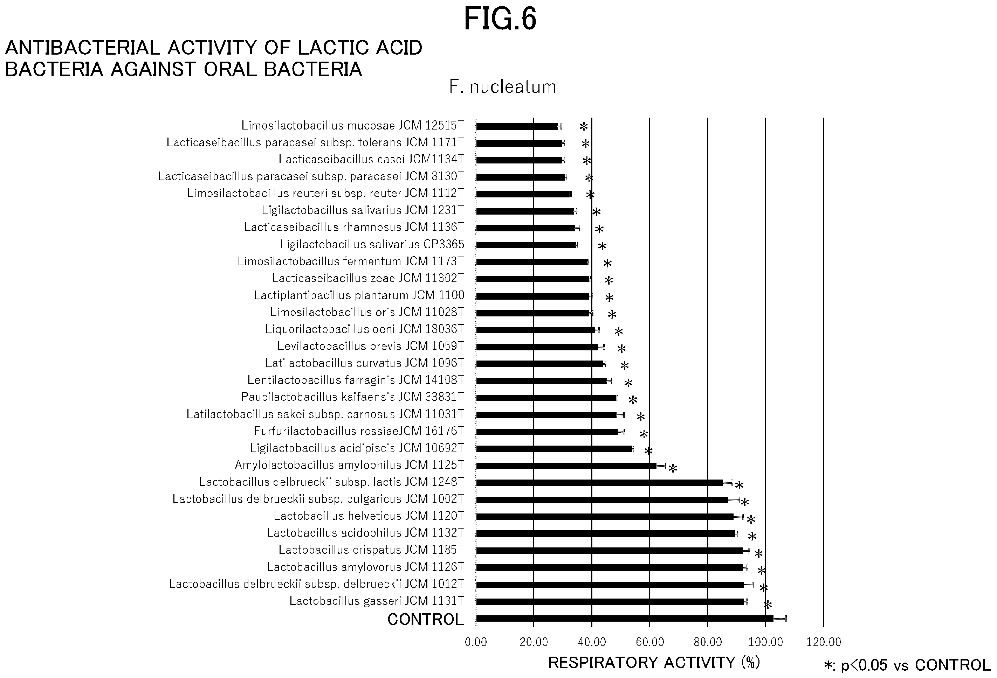

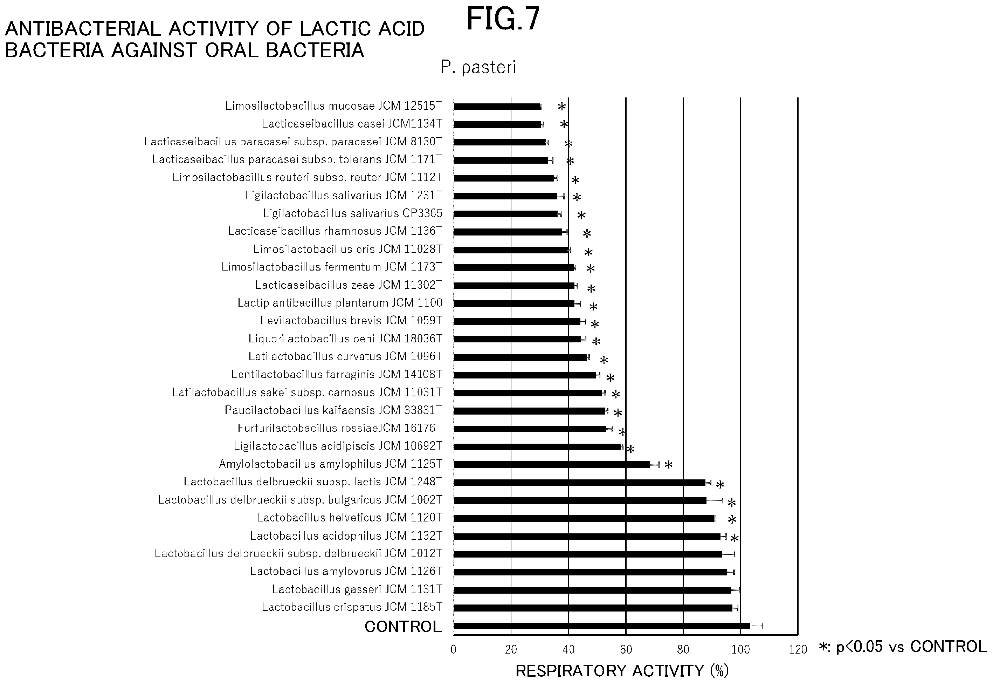

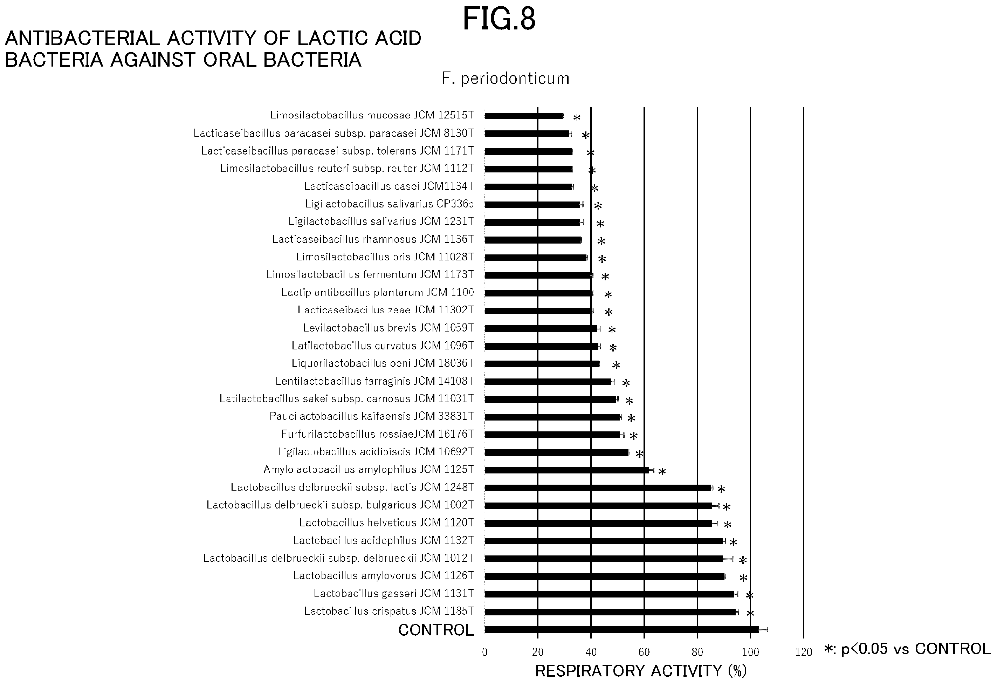

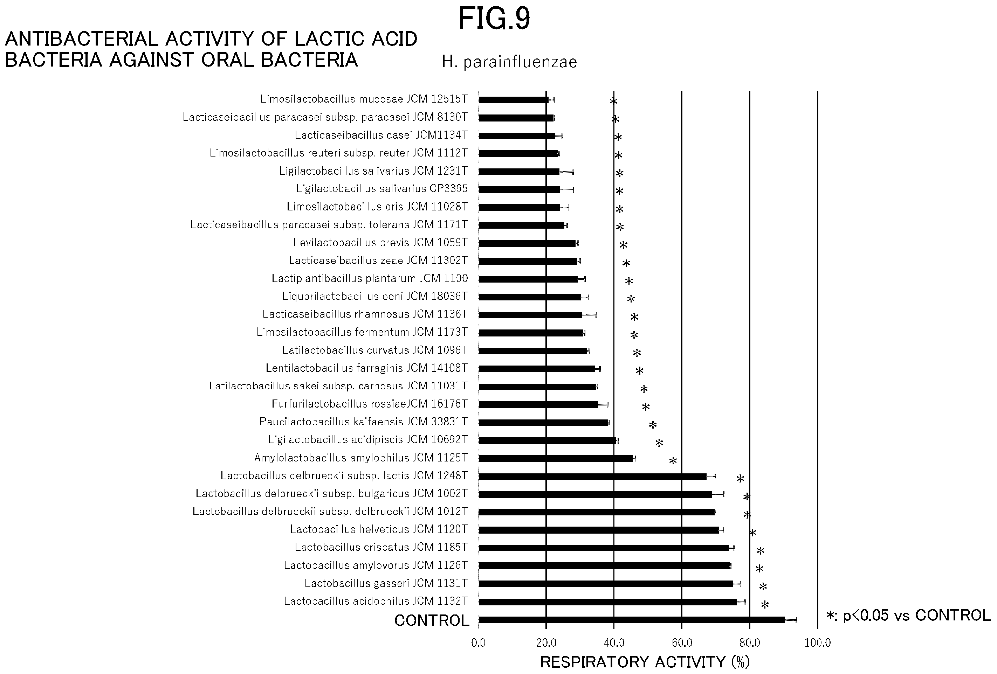

Example 4 was carried out to evaluate the antibacterial activity of the killed bacteria of the lactic acid bacteria strains specified in Table 1, other than *Ligilactobacillus salivarius,* against the five species of the oral bacteria targeted for the evaluations in Examples 1 and 3. The evaluation was carried out according to the same procedure as in Example 1. The antibacterial activity of the CP3365 strain and the JCM1231^{T} strain of *Ligilactobacillus salivarius* were also evaluated. Figs. 5 to 9 show the results.

Among the killed lactic acid bacteria other than *Ligilactobacillus salivarius,* killed lactic acid bacteria that significantly inhibited the respiratory activity of the five species of oral bacteria were also found (Figs. 5 to 9). In particular, the killed lactic acid bacteria other than the genus *Lactobacillus* significantly inhibited the respiratory activity of all the five species of oral bacteria.

Moreover, the antibacterial activity of the killed lactic acid bacteria of the genus *Limosilactobacillus* and the genus *Lacticaseibacillus* tended to be higher than the antibacterial activity of the killed bacteria of *Ligilactobacillus salivarius.*

### [Industrial Applicability]

The present invention is usable in foods and/or beverages and others.

**Table 1: Test Bacterial Strains**

| Type | Bacteria Name | Strain Number | Depository | Quantity of Collected Bacteria (mg) |
|---|---|---|---|---|
| Lactic Acid Bacteria | Amylolactobacillus amylophilus | JCM 1125T | RIKEN-BRC JCM | 7.8 |
| | Furfurilactobacillus rossiae | JCM 16176T | RIKEN-BRC JCM | 48.0 |
| | Lacticaseibacillus casei | JCM 1134T | RIKEN-BRC JCM | 51.8 |
| | Lacticaseibacillus paracasei subsp. paracasei | JCM 8130T | RIKEN-BRC JCM | 49.8 |
| | Lacticaseibacillus paracasei subsp. tolerans | JCM 1171T | RIKEN-BRC JCM | 32.0 |
| | Lacticaseibacillus rhamnosus | JCM 1136T | RIKEN-BRC JCM | 108.2 |
| | Lacticaseibacillus zeae | JCM 11302T | RIKEN-BRC JCM | 76.0 |
| | Lactiplantibacillus plantarum | JCM 1100 | RIKEN-BRC JCM | 73.2 |
| | Lactobacillus acidophilus | JCM 1132T | RIKEN-BRC JCM | 55.0 |
| | Lactobacillus amylovorus | JCM 1126T | RIKEN-BRC JCM | 84.6 |
| | Lactobacillus crispatus | JCM 1185T | RIKEN-BRC JCM | 35.0 |
| | Lactobacillus delbrueckii subsp. bulgaricus | JCM 1002T | RIKEN-BRC JCM | 37.0 |
| | Lactobacillus delbrueckii subsp. delbrueckii | JCM 1012T | RIKEN-BRC JCM | 50.8 |
| | Lactobacillus delbrueckii subsp. lactis | JCM 1248T | RIKEN-BRC JCM | 31.2 |
| | Lactobacillus gasseri | JCM 1131T | RIKEN-BRC JCM | 74.8 |
| | Lactobacillus helveticus | JCM 1120T | RIKEN-BRC JCM | 45.2 |
| | Latilactobacillus curvatus | JCM 1096T | RIKEN-BRC JCM | 35.0 |
| | Latilactobacillus sakei subsp. carnosus | JCM 11031T | RIKEN-BRC JCM | 28.2 |
| | Lentilactobacillus farraginis | JCM 14108T | RIKEN-BRC JCM | 46.0 |
| | Levilactobacillus brevis | JCM 1059T | RIKEN-BRC JCM | 21.2 |
| | Ligilactobacillus acidipiscis | JCM 10692T | RIKEN-BRC JCM | 21.2 |
| | Ligilactobacillus salivarius | CP 3365 | ASAHI | 129.4 |
| | Ligilactobacillus salivarius | JCM 1231T | RIKEN-BRC JCM | 117.6 |
| | Ligilactobacillus salivarius | JCM 1040T | RIKEN-BRC JCM | 53.0 |
| | Ligilactobacillus salivarius | JCM 1150T | RIKEN-BRC JCM | 110.8 |
| | Ligilactobacillus salivarius | JCM 7706T | RIKEN-BRC JCM | 102.8 |
| | Ligilactobacillus salivarius | JCM 7712T | RIKEN-BRC JCM | 89.6 |
| | Limosilactobacillus fermentum | JCM 1173T | RIKEN-BRC JCM | 12.4 |
| | Limosilactobacillus mucosae | JCM 12515T | RIKEN-BRC JCM | 54.4 |
| | Limosilactobacillus oris | JCM 11028T | RIKEN-BRC JCM | 40.4 |
| | Limosilactobacillus reuteri subsp. reuteri | JCM 1112T | RIKEN-BRC JCM | 67.6 |
| | Liquorilactobacillus oeni | JCM 18036T | RIKEN-BRC JCM | 67.0 |
| | Paucilactobacillus kaifaensis | JCM 33831T | RIKEN-BRC JCM | 15.4 |
| Oral Bacteria | Fusobacterium nucleatum | JCM 8532T | RIKEN-BRC JCM | - |
| | Fusobacterium periodonticum | JCM 12991 T | RIKEN-BRC JCM | - |
| | Haemophilus parainfluenzae | ATCC 33392T | ATCC | - |
| | Porphyromonas gingivalis | ATCC 33277T | ATCC | - |
| | Porphyromonas pasteri | JCM 30531T | RIKEN-BRC JCM | - |

**Table 2: Clinical Trial Results**

| Indicator | Group | Δ Week 4 | | | | Δ Week 8 | | | |
|---|---|---|---|---|---|---|---|---|---|
| | | Average | | SE | p value | Average | | SE | p value |
| PCR (%) | Control Food | 4.15 | ± | 1.44 | 0.02 | 6.00 | ± | 1.61 | 0.02 |
| | CP3365 | -1.06 | ± | 1.50 | | 0.44 | ± | 1.68 | |
| BOP (%) | Control Food | 2.36 | ± | 0.61 | 0.10 | 0.91 | ± | 0.28 | 0.02 |
| | CP3365 | 0.86 | ± | 0.34 | | -0.15 | ± | 0.32 | |
| PPD (mm) | Control Food | 0.04 | ± | 0.02 | 0.37 | 0.10 | ± | 0.03 | 0.05 |
| | CP3365 | 0.00 | ± | 0.02 | | 0.02 | ± | 0.03 | |

| | | | | | | | | | |
|---|---|---|---|---|---|---|---|---|---|
| p value: Control Food Group vs CP3365 | | | | | | | | | |

**[Table 3]**

| Table 3: Changes of Oral Bacterial Floras in Plaque and Salvia a) Plaque | | | | | | | | | | | | | |
|---|---|---|---|---|---|---|---|---|---|---|---|---|---|
| Species | Group | Week 0 | | | | | Week 8 | | | | | | |
| | | Occupancy Rate | | | Detection Count* | | Occupancy Rate | | | Detection Count* | Change (8w-0w) | | |
| | | Average | SE | p value 1) | n= | Average | SE | p value 1) | p value 2) | n= | Average | SE | p value 1) |
| Porphyromonas gingivalis | Control Food | 0.20 | 0.20 | 1.000 | 1 | 0.20 | 0.20 | 1.000 | 1.000 | 1 | 0.00 | 0.40 | 0.967 |
| | CP3365 | 0.57 | 0.47 | | 2 | 0.20 | 0.10 | | 0.414 | 3 | -0.45 | 0.55 | |
| Porphyromonas pasteri | Control Food | 3.63 | 0.66 | 0.954 | 21 | 4.71 | 1.03 | 0.377 | 0.132 | 25 | 1.11 | 0.56 | 0.061 |
| | CP3365 | 3.74 | 0.75 | | 22 | 3.09 | 0.48 | | 0.231 | 24 | -0.66 | 0.51 | |
| Fusobacterium nucleatum subsp. animalis | Control Food | 0.97 | 0.58 | 1.000 | 3 | 1.97 | 0.65 | 1.000 | 0.109 | 3 | 1.00 | 0.47 | 0.199 |
| | CP3365 | 1.00 | 1.00 | | 2 | 1.55 | 0.25 | | 0.655 | 2 | 0.50 | 1.30 | |
| Fusobacterium nucleatum subsp. vincentii | Control Food | 0.48 | 0.30 | 0.556 | 2 | 0.78 | 0.32 | 0.905 | 0.416 | 3 | 0.30 | 0.61 | 0.739 |
| | CP3365 | 0.70 | 0.31 | | 3 | 0.63 | 0.40 | | 1.000 | 2 | -0.08 | 0.69 | |
| Fusobacterium periodonticum | Control Food | 1.97 | 029 | 0.925 | 17 | 1.54 | 0.25 | 0.925 | 0227 | 16 | -0.43 | 0.34 | 0.821 |
| | CP3365 | 2.04 | 0.30 | | 16 | 1.68 | 0.36 | | 0.289 | 13 | -0.36 | 0.43 | |
| Haemophilus parainfluenzae | Control Food | 2.56 | 0.41 | 0.317 | 21 | 2.59 | 0.47 | 0.198 | 0.853 | 20 | 0.02 | 0.37 | 0.567 |
| | CP3365 | 2.96 | 0.36 | | 21 | 3.15 | 0.36 | | 0.578 | 21 | 0.20 | 0.43 | |

| | | | | | | | | | | | | | |
|---|---|---|---|---|---|---|---|---|---|---|---|---|---|
| p value 1): Inter-Group Difference, p value 2): Intra-Group Difference * Detection Count: Control Food Group n=25, CP3365 Group n=25 | | | | | | | | | | | | | |

### b) Salvia

| Species | Group | Week 0 | | | | Week 8 | | | | Detection Count* | Change (8w-0w) | | |
|---|---|---|---|---|---|---|---|---|---|---|---|---|---|
| | | Occupancy Rate | | | Detection Count* | Occupancy Rate | | | | | | | |
| | | Average | SE | p value 1) | n= | Average | SE | p value 1) | p value 2) | n= | Average | SE | p value 1) |
| Porphyromonas gingivalis | Control Food | 0.27 | 0.04 | 0.740 | 7 | 0.26 | 0.09 | 0.536 | 0.492 | 6 | -0.04 | 0.14 | 0.087 |
| | CP3365 | 027 | 0.07 | | 8 | 054 | 0.25 | | 0227 | 10 | 0.39 | 0.25 | |
| Porphyromonas pasteri | Control Food | 664 | 0.48 | 0.137 | 27 | 4.87 | 0.41 | 0.135 | 0.000 | 27 | -1.78 | 0.38 | 0.444 |
| | CP3365 | 5.37 | 0.46 | | 26 | 3.87 | 0.38 | | 0.000 | 26 | -1.60 | 0.35 | |
| Fusobacterium nucleatum subsp. anmalis | Control Food | 0.00 | 0.00 | - | 0 | 0.00 | 0.00 | - | - | 0 | 0 | | - |
| | CP3365 | 0.00 | 0.00 | | 0 | 0.00 | 0.00 | | - | 0 | 0 | | |
| Fusobacterium nucleatum subsp. vincentii | Control Food | 053 | 0.26 | 0.860 | 5 | 0.74 | 0.30 | 0.659 | 0.497 | 5 | 0.21 | 0.30 | 0.554 |
| | CP3365 | 0.43 | 0.15 | | 6 | 0.82 | 0.16 | | 0.092 | 11 | 0.49 | 0.27 | |
| Fusobacterium periodonticum | Control Food | 7.71 | 0.79 | 0.089 | 26 | 7.43 | 0.83 | 0.032 | 0.770 | 27 | -0.29 | 0.64 | 0.510 |
| | CP3365 | 585 | 0.69 | | 26 | 4.95 | 0.67 | | 0.174 | 25 | -0.88 | 0.53 | |
| Haemophilus parainfluenzae | Control Food | 3.91 | 0.37 | 0.285 | 26 | 4.24 | 0.27 | 0.032 | 0.301 | 27 | 0.33 | 0.35 | 0.305 |
| | CP3365 | 3.37 | 0.23 | | 26 | 3.38 | 0.26 | | 0.976 | 26 | 0.00 | 0.22 | |

| | | | | | | | | | | | | | |
|---|---|---|---|---|---|---|---|---|---|---|---|---|---|
| p value 1): Inter-Group Difference, p value 2): Intra-Group Difference *Detecion Count: Control Food Group n=27, CP3365 Group n=26 | | | | | | | | | | | | | |

## Claims

1. A composition for maintaining an oral environment or gum health, comprising a killed lactic acid bacterium as an active ingredient, wherein
the killed bacterium inhibits plaque maturation in an oral cavity.

2. The composition according to claim 1, wherein the killed bacterium inhibits the plaque maturation in the oral cavity by inhibiting proliferation of at least one species of oral bacteria selected from the group consisting of
*Porphyromonas pasteri,*
*Fusobacterium periodonticum,* and
*Haemophilus parainfluenzae.*

3. The composition according to claim 1, wherein the killed bacterium inhibits the plaque maturation in the oral cavity by reducing an occupancy rate, in an oral bacterial flora, of at least one species of oral bacteria selected from the group consisting of
*Porphyromonas pasteri,*
*Fusobacterium periodonticum,* and
*Haemophilus parainfluenzae.*

4. The composition according to claim 1, wherein the killed bacterium is a killed lactic acid bacterium selected from the group consisting of
the genus *Amylolactobacillus,*
the genus *Furfurilactobacillus,*
the genus *Lacticaseibacillus,*
the genus *Lactiplantibacillus,*
the genus *Latilactobacillus,*
the genus *Lentilactobacillus,*
the genus *Levilactobacillus,*
the genus *Ligilactobacillus,*
the genus *Limosilactobacillus,*
the genus *Liquorilactobacillus,* and
the genus *Paucilactobacillus.*

5. The composition according to claim 1, wherein the killed bacterium is a killed lactic acid bacterium selected from the group consisting of
*Amylolactobacillus amylophilus,*
*Furfurilactobacillus rossiae,*
*Lacticaseibacillus casei,*
*Lacticaseibacillus paracasei* subsp. *paracasei,*
*Lacticaseibacillus paracasei* subsp. *tolerans,*
*Lacticaseibacillus rhamnosus,*
*Lacticaseibacillus zeae,*
*Lactiplantibacillus plantarum,*
*Latilactobacillus curvatus,*
*Latilactobacillus sakei* subsp. *carnosus,*
*Lentilactobacillus farraginis,*
*Levilactobacillus brevis,*
*Ligilactobacillus acidipiscis,*
*Ligilactobacillus salivarius,*
*Limosilactobacillus fermentum,*
*Limosilactobacillus mucosae,*
*Limosilactobacillus oris,*
*Limosilactobacillus reuteri* subsp. *reuteri,*
*Liquorilactobacillus oeni,* and
*Paucilactobacillus kaifaensis.*

6. The composition according to claim 1, wherein the killed bacterium is a killed bacterium of *Ligilactobacillus salivarius.*

7. The composition according to claim 1, wherein the killed bacterium is a killed bacterium of a CP3365 strain (accession number: NITE BP-03781) of *Ligilactobacillus salivarius.*

8. The composition according to claim 1, wherein the maintaining the oral environment is inhibiting deterioration of plaque control record (PCR).

9. The composition according to claim 1, wherein the maintaining the gun health is inhibiting deterioration of probing pocket depth (PPD).

10. The composition according to claim 1, wherein the maintaining the gum health is inhibiting deterioration of bleeding on probing (BOP).

11. The composition according to claim 1, which is for a healthy individual.

12. The composition according to claim 1, which is a food and/or beverage composition.

13. The composition according to claim 12, which is in a tablet form.

14. The composition according to claim 12, which is in a beverage form.

15. A CP3365 strain (accession number: NITE BP-03781) of *Ligilactobacillus salivarius* or a killed bacterium of the strain.
